# EUROPÄISCHE PATENTSCHRIFT

(11) **EP 3 919 550 B1**
(45) Veröffentlichungstag und Bekanntmachung des Hinweises auf die Patenterteilung: **07.05.2025**
(21) Anmeldenummer: 20177746.3
(22) Anmeldetag: 02.06.2020
(51) Int. Cl.: C08G 77/14, C08K 5/09, C08G 77/46, C08L 83/12

(54) **LINEARE ACETOXYGRUPPEN-TRAGENDE SILOXANE UND FOLGEPRODUKTE**
LINEAR ACETOXY GROUP BEARING SILOXANES AND SECONDARY PRODUCTS
SILOXANES LINÉAIRES TRANSPORTANT DES GROUPES ACETOXY ET PRODUITS DÉRIVÉS

(43) Veröffentlichungstag der Anmeldung: 08.12.2021
(73) Patentinhaber: Evonik Operations GmbH, 45128 Essen (DE)
(72) Erfinder: KNOTT, Wilfried, 45355 Essen (DE); DUDZIK, Horst, 45326 Essen (DE); FAVRESSE, Philippe, 40880 Ratingen (DE)
(74) Vertreter: Evonik Patent Association

(56) Entgegenhaltungen:
- WO-A1-2019/219446
- CA-A- 1 125 780

## Beschreibung

Die Erfindung liegt auf dem Gebiet der Silikonchemie. Sie betrifft ein Verfahren zur Herstellung linearer Acetoxygruppen-tragender Siloxane und die nach dem Verfahren hergestellten supersauren (end)äquilibrierten linearen α,ω-Acetoxygruppen-tragenden Siloxane.

Zugänge zu Acetoxy-funktionellen Siloxanen sind in der Literatur bereits beschrieben. So ist die nicht äquilibrierende Öffnung einfacher unverzweigter Siloxanzyklen mit Acetanhydrid zu kurzkettigen, kettenterminale Acetoxygruppen tragenden Siloxanen in Gegenwart von Katalysatoren bekannt.

Borisov und Sviridova beschreiben die Öffnung zyklischer Dimethylsiloxane mit Acetanhydrid in Gegenwart katalytischer Mengen Eisen(III)chlorid zu kurzkettigen α,ω-Acetoxysiloxanen ( S. N. Borisov, N. G.Sviridova, J. Organomet. Chem. 11 (1968), 27-33 ). Lewis et al. widmet sich in der US 4,066,680 der Herstellung kurzkettiger α,ω-Siloxandiole, wobei er Octamethylcyclotetrasiloxan mit Acetanhydrid an säurebehandelten Bleicherden umsetzt und die so erhaltenen Mischungen kurzkettiger α,ω-Acetoxysiloxane in alkalisch eingestelltem Wasser hydrolysiert.

Aus US 3,346,610 ist gleichfalls ein Zugang zu Acetoxygruppen tragenden, kurzkettigen Siloxanen bekannt, der auf der Metallhalogenid induzierten Acetoxy-Modifizierung gespannter cyclischer Siloxane beruht, indem man diese mit Acetoxygruppen enthaltenden Silikonverbindungen zur Umsetzung bringt. Eine Vielzahl Friedel-Crafts-aktiver Metallhalogenide fungiert hier als Katalysator, wobei Zinkchlorid als bevorzugt ausgelobt wird. Eine spezielle Zielsetzung der US 3,3466,10 liegt in der Acetoxy-Modifizierung gespannter Diorganosiloxancyclen unter bewusster Vermeidung von Äquilibriervorgängen.

Der Stand der Technik bezieht sich somit auf Arbeiten, die die Öffnung cyclischer Siloxane - hierbei manchmal gespannter Cyclosiloxane - mit Acyloxygruppen enthaltenden Reaktanden vorsehen und deren Zielsetzung es ist, definierte lineare kurzkettige und das auf dem Wege der fraktionierten Destillation noch zu separierende Siloxanspezies zu gewinnen.

Jedoch sind die auf diesem Wege synthetisierten, Molmassen-definierten, kettenreinen Acetoxymodifizierten Siloxanverbindungen nicht geeignet für die Herstellung von organomodifizierten Siloxanen insbesondere Polyethersiloxanen, die Eingang in anspruchsvolle technische Anwendungen wie z.B. in die PU-Schaumstabilisierung oder in die Entschäumung von Kraftstoffen, etc. nehmen. Wirkstoffe, die ein derartiges Anwendungsgebiet effektiv adressieren, sind stets von einer breiten Oligomerverteilung umfassend hohe, mittlere und niedrige Molmassen gekennzeichnet, da den darin enthaltenen Oligomeren in Abhängigkeit von ihrer Molmasse und damit ihres Diffusionsverhaltens sehr oft differenzierte tensidische Aufgaben in unterschiedlichen Zeitfenstern des jeweiligen Prozesses zuzuschreiben sind.

Ebenfalls sind Acyloxy-organopolysiloxane und hierbei insbesondere Organosiloxane mit endständigen Acyloxygruppen als Ausgangsmaterialien für Folgereaktionen bekannt. So können beispielsweise die Acyloxygruppen an einem Diorganosiloxan hydrolysiert werden, worauf das Hydrolysat dehydratisiert und das dehydratisierte Hydrolysat unter Bildung von fließfähigem Diorganopolysiloxan polymerisiert werden kann. Diese fließfähigen Polysiloxane eignen sich als Ausgangsmaterialien für die Herstellung viskoser Öle und Kautschuke, die zu Silikonelastomeren gehärtet werden können.

Mit endständigen Acyloxygruppen versehene Organosiloxane können beispielsweise durch Umsetzung eines Alkylsiloxans und einer organischen Säure und/oder deren Anhydrid in Gegenwart von Schwefelsäure als Katalysator erhalten werden. Ein solches Verfahren ist in der US-Patentschrift 2 910 496 (Bailey et al.) beschrieben. Obwohl man nach diesem Verfahren prinzipiell auch Organosiloxane mit endständigen Acyloxygruppen erhält, so haftet dem Prozess der Nachteil an, dass das Reaktionsprodukt aus einer Mischung von acyloxyhaltigen Siloxanen und Acyloxygruppen tragenden Silanen unterschiedlicher Zusammensetzung besteht. Insbesondere führt die Lehre hierzu aus, dass aus M-, D- und T-Einheiten zusammengesetzte Alkylsiloxancopolymere durch das Verfahren in Trimethyl-acyloxysilan, Di-Acyloxydimethylsiloxan und Methyltriacyloxysilan gespalten werden. Somit erhält Bailey bei der Umsetzung von Octamethylcyclotetrasiloxan mit Essigsäureanhydrid und Essigsäure nach Neutralisation der als Katalysator eingesetzten Schwefelsäure, Abtrennen der Salze und Abziehen von Wasser, restlicher Essigsäure und Acetanhydrids ein komplexes Stoffgemisch und keinesfalls ein Äquilibrat, das er dann der fraktionierten Destillation unterwirft (siehe Beispiel, ibid.). Die stoffliche Identität der dabei erhaltenen Fraktionen II und IV bleibt unklar, so dass es hiernach schwierig ist, definierte Produkte zu erhalten, beziehungsweise diese in hohen Ausbeuten vom Gemisch abzutrennen.

Sich auf Bailey et al. (US 2 910 496 ) beziehend, lehrt die DE-OS 1545110 (A1) (Omietanski et al. ) ein Verfahren, bei dem eine Acyloxygruppe eines Acyloxysiloxans mit der Hydroxylgruppe eines Polyoxyalkylenhydroxypolymers unter Bildung eines Siloxan-oxyalkylen-Blockmischpolymers und einer Carbonsäure umgesetzt wird, wobei die Carbonsäure aus dem Reaktionsgemisch entfernt wird. Die dort beschriebenen, lösemittel- und katalysatorfrei geführten Umsetzungen verlangen zum Teil beträchtliche Reaktionszeiten (bis zu 11,5 Stunden (Beispiel 1), sehr hohe, produktbelastende Umsetzungstemperaturen (150 bis 160°C (Beispiel 1) und das Anlegen eines Hilfsvakuums beziehungsweise das Strippen der Reaktionsmatrix mit trockenem Stickstoff über die gesamte Reaktionsdauer und erreichen trotz der harschen Umsetzungsbedingungen auf Produktstufe nicht immer vollständigen Umsatz (Beispiel 9, ibid.).

Aus produktionstechnischer Sicht gereichen insbesondere die Kombination aus hohen Umsetzungstemperaturen und langen Reaktionszeiten sowie die nicht vorhersehbare Produktqualität dem von Omietanski et al. beschriebenen Prozess zum Nachteil.

Die Lehre der Anmeldung EP 3611217 A1 offenbart, dass man trifluormethansulfonsaure äquilibrierte α,ω-Diacetoxysiloxane durch die Umsetzung von Siloxancyclen (D₄ und/oder D₅) mit Acetanhydrid in Gegenwart von Trifluormethansulfonsäure herstellen kann und dass diese Diacetoxysiloxane mit Polyether(mono)olen bei mäßigen Temperaturen zügig und zudem vollständig zu SiOC-verknüpften Polyethersiloxanen des Strukturtyps ABA reagieren.

CA1125780 A offenbart ein Verfahren zur Herstellung von α,ω-Siloxandiolen aus α,ω-Diacetoxysiloxanen als Zwischenprodukt. Die α,ω-Diacetoxysiloxane werden durch Reaktion von Siloxandiolen mit Acetatanhydrid und Essigsäure in Gegenwart einer sauren Bleicherde unter Rückfluss hergestellt.

Die Erfinder haben nun überraschenderweise gefunden, dass man auch zu supersauren (vorzugsweise trifluormethan-sulfonsauren), endäquilibrierten α,ω-Diacetoxysiloxanen, und zwar insbesondere zu solchen wie in EP 3611217 A1 und EP 3611216 A1beschrieben, gelangt, indem man lineare Hydroxygruppen tragende Siloxane mit Essigsäureanhydrid, Supersäure (vorzugsweise Perfluoralkansulfonsäure, insbesondere Trifluormethansulfonsäure) sowie Essigsäure zur Umsetzung bringt.

Gegenstand der vorliegenden Erfindung ist somit ein Verfahren zur Herstellung supersaurer, vorzugsweise trifluormethansulfonsaurer, (end)äquilibrierter linearer α,ω-Acetoxygruppen-tragender Siloxane, wobei man
(i) lineare α,ω-Hydroxygruppen tragende Siloxane,
(ii) unter Einsatz von Supersäure, bevorzugt Perfluoralkansulfonsäure, besonders bevorzugt Trifluormethansulfonsäure als Katalysator
(iii) mit Acetanhydrid und unter Zusatz von Essigsäure
umsetzt.

Die erfindungsgemäß resultierenden supersauren, endäquilibrierten linearen α,ω-Diacetoxysiloxane, hergestellt nach dem erfindungsgemäßen Verfahren, weisen eine derartig hohe Reaktivität auf, dass man sie zum Beispiel mit Polyetherolen, Polyetherdiolen und/oder Monoolen zu den anspruchsvollen linearen SiOC-verknüpften Polyethersiloxan-Strukturen weiterverarbeiten kann.

Die resultierenden supersauren, endäquilibrierten linearen α,ω-Diacetoxysiloxane können zur Herstellung von linearen SiOC-verknüpften Polyethersiloxanen verwendet werden.

Supersaure (vorzugsweise trifluormethansulfonsaure) endäquilibrierte lineare α,ω-Diacetoxypolydimethylsiloxane sind erfindungsgemäß dadurch erhältlich, dass man Hydroxygruppen tragende Siloxane unter Einsatz von Supersäure (vorzugsweise Perfluoralkansufonsäure, insbesondere Trifluormethansulfonsäure) als Katalysator mit Acetanhydrid und unter Zusatz von Essigsäure umsetzt.

Die Supersäure (vorzugsweise Trifluormethansulfonsäure) wird dabei vorzugsweise in Mengen von 0,1 bis 1,0 Gewichtsprozent, bevorzugt 0,1 bis 0,3 Gewichtsprozent, bezogen auf die Acetanhydrid und Hydroxygruppen tragende Siloxane umfassende Reaktionsmatrix, eingesetzt. Die Umsetzung wird vorzugsweise im Temperaturbereich von 140 bis 160°C und vorzugsweise in einem Zeitraum von 4 bis 8 Stunden durchgeführt.

Vorzugsweise genügen die erfindungsgemäßen linearen α,ω-Hydroxygruppen tragenden Siloxane zumindest der Formel (I) 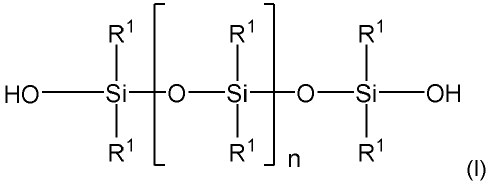 mit R¹ gleich Alkylrest und/oder aromatischer Rest, umfassend 1 bis 10 C-Atome, bevorzugt ein Methylrest und mit 1 ≤ n ≤ 19.000, bevorzugt n zwischen 3 und 200, besonders bevorzugt n zwischen 20 und 100.

Erfindungsgemäß werden unter dem Begriff "Supersäure" Brönstedtsäuren verstanden, das heißt, Protonen liefernde Verbindungen. Als Supersäure kommen somit sowohl homogene als auch heterogene, sowohl flüssige als auch feste und hierunter besonders auch polymere und/ oder trägerfixierte saure Systeme in Betracht. Umfasst sind auch Heteropolysäuren. Zusammen mit aziden Wasserstoffionen bilden Polyoxometallate Heteropolysäuren, welche als Säuren im Sinne der Erfindung eingesetzt werden können. Die Säuren fungieren erfindungsgemäß als Katalysatoren.

Der Katalysator Supersäure, bevorzugt Perfluoralkansulfonsäure, besonders Trifluormethansulfonsäure, wird gemäß einer bevorzugten Ausführungsform der Erfindung in Mengen von 0,1 bis 1,0 Gewichtsprozent, bevorzugt 0,1 bis 0,3 Gewichtsprozent, bezogen auf die Acetanhydrid und Hydroxygruppen tragende Siloxane umfassende Reaktionsmatrix, eingesetzt.

Gemäß einer bevorzugten Ausführungsform der Erfindung setzt man Essigsäure in Mengen von 0,4 bis 3,5 Gewichtsprozent, vorzugsweise 0,5 bis 3 Gewichtsprozent, bevorzugt 0,8 bis 1,8 Gewichtsprozent, besonders bevorzugt in Mengen von 1,0 bis 1,5 Gewichtsprozent bezogen auf die Reaktionsmatrix umfassend Acetanhydrid und Hydroxygruppen tragende Siloxane, hinzu.

Gemäß einer bevorzugten Ausführungsform der Erfindung ist die einzusetzende Acetanhydridmenge mindestens so zu bemessen, das alle Si-gebundenen Hydroxygruppen des eingesetzten α,ω-Hydroxygruppen tragenden Siloxans durch Acetoxygruppen ersetzt werden und gleichzeitig das freigesetzte Wasseräquivalent durch Reaktion mit weiterem Acetanhydrid in Form von zwei Äquivalenten Essigsäure gebunden wird.

Gemäß einer besonders bevorzugten Ausführungsform erfolgt die Umsetzung in einem Reaktor, dessen Volumen mindestens 1 Liter, vorzugsweise mindestens 5 Liter, insbesondere mindestens 10 Liter beträgt, und vorzugsweise maximal 500000 Liter beträgt.

Der Begriff des Reaktors ist dem Fachmann bekannt. Als Reaktor wird ein abgegrenzter Raum, beispielsweise ein gerührter Behälter oder ein Rohr bezeichnet, in dem chemische Stoffumwandlungen gezielt durchgeführt werden können. Es können dies, wie der Fachmann weiß, offene oder geschlossene Behälter sein, in denen die Edukte in die gewünschten Produkte oder Zwischenprodukte umgewandelt werden. Das Volumen von Reaktoren wird vom Hersteller angegeben oder kann durch Auslitern bestimmt werden.

Neben den erfindungsgemäß obligatorisch einzusetzenden linearen α,ω-Hydroxygruppen tragenden Siloxanen können optional auch Hydroxygruppen tragende Silane, wie z.B. Dimethylsilandiol, und/ oder einfache Siloxanzyklen , insbesondere umfassend D₄ und/ oder D₅, zusätzlich eingesetzt werden und können Teil der Reaktionsmatrix sein.

Die supersauren, vorzugsweise trifluormethansulfonsauren, (end)äquilibrierten linearen α,ω-Acetoxygruppen-tragenden Siloxane weisen im Sinne einer bevorzugten Ausführungsform der Erfindung zumindest 3, vorzugsweise 5 bis 50, bevorzugt 7 bis 25, besonders bevorzugt 10 bis 20 Organosiloxaneinheiten auf.

Erfindungsgemäß besonders bevorzugt sind trifluormethansulfonsaure endäquilibrierte lineare α,ω-Diacetoxypolydimethylsiloxane.

Mit dem Begriff "endäquilibriert" ist gemeint, dass das Äquilibriergleichgewicht erreicht worden, das sich bei einer Temperatur von 23°C und einem Druck von 1013,25 hPa einstellt. Als Indikator für das Erreichen des Äquilibriergleichgewichtes kann der gaschromatographisch bestimmte Gesamtcylengehalt definiert als die Summe der D₄-, D₅-, D₆-Gehalte bezogen auf die Siloxanmatrix und festgestellt nach der Derivatisierung der α,ω-Diacetoxypolydimethylsiloxane zu den entsprechenden α,ω-Diisopropoxypolydimethylsiloxanen herangezogen werden. Der erfindungsgemäße Einsatz der Essigsäure ermöglicht hier die problemlose Unterschreitung sonst üblicher Gleichgewichtsanteile von etwa 13 Gewichtsprozent des Gesamtcyclengehaltes bei den linearen α,ω-Diacetoxypolydimethylsiloxanen. Demzufolge entspricht es einer bevorzugten Ausführungsform wenn Gleichgewichtsanteile des Gesamtcyclengehaltes von kleiner 13, vorzugsweise kleiner 12 Gewichtsprozent bei den linearen α,ω-Diacetoxypolydimethylsiloxanen, unterschritten werden. Die Derivatisierung zu den α,ω-Diisopropoxypolydimethylsiloxanen wird hierbei bewusst gewählt, um eine unter den Bedingungen der gaschromatographischen Analyse gegebenenfalls erfolgende, thermisch induzierte Rückspaltungsreaktion der α,ω-Diacetoxypolydimethylsiloxane zu verhindern (zur Rückspaltungsreaktion siehe u.a. J. Pola et al., Collect. Czech. Chem. Commun. 1974, 39(5), 1169-1176 und auch W. Simmler, Houben-Weyl, Methods of Organic Chemistry, Vol. VI/2, 4th Edition, O-Metal Derivates of Organic Hydroxy Compounds S. 162 ff)).

Wie aufgezeigt, ermöglicht das erfindungsgemäße Verfahren den eleganten Zugang zu supersauren, vorzugsweise trifluormethansulfonsauren, endäquilibrierten linearen α,ω-Acetoxygruppen-tragende Siloxanen. Ein weiterer Gegenstand der vorliegenden Erfindung sind demnach supersaure, vorzugsweise trifluormethansulfonsaure, endäquilibrierte lineare α,ω-Acetoxygruppen-tragende Siloxane, hergestellt nach einem Verfahren wie zuvor beschrieben, welche sich dadurch auszeichnen, dass sie Gesamtcyclengehalte, definiert als Summe der Gehaltsanteile der zyklischen Siloxane umfassend D₄, D₅ und D₆ bezogen auf die Siloxanmatrix und gaschromatographisch ermittelt nach deren Derivatisierung zu den entsprechenden linearen α,ω-Isopropoxysiloxanen von kleiner 13, vorzugsweise kleiner 12 Gewichtsprozent aufweisen. Gemäß einer bevorzugten Ausführungsform weisen die supersauren, vorzugsweise trifluormethansulfonsauren, (end)äquilibrierten linearen α,ω-Acetoxygruppen-tragenden Siloxane zumindest 3, vorzugsweise 5 bis 50, bevorzugt 7 bis 25, besonders bevorzugt 10 bis 20 Organosiloxaneinheiten auf.

Die erfindungsgemäß erhältlichen endäquilibrierten supersauren, vorzugsweise trifluormethansufonsauren linearen Acetoxygruppen-tragenden Siloxane können als Ausgangsmaterialien zur Herstellung von SiOC-verknüpften linearen Polyethersiloxanen verwendet werden.

Die endäquilibrierten supersauren, vorzugsweise trifluormethansufonsauren linearen Acetoxygruppen-tragenden Siloxane finden Anwendung als Ausgangsmaterialien zur Herstellung von linearen SiOC-verknüpften Polyethersiloxanen, insbesondere für deren nachfolgenden Einsatz in PU-Schaumstabilisatoren, in Entschäumern, in Dismulgatoren, in Emulgatoren und in Lack- und Verlaufsadditiven; sowie für deren Einsatz als Entlüfter; als Schaumstabilisator, insbesondere als Polyurethanschaumstabilisator; als Netzmittel; als Hydrophobierungsmittel; als Verlaufsmittel; zur Herstellung von Polymerdispersionen; zur Herstellung von Klebstoffen oder Dichtstoffen; zur Oberflächenbehandlung von Fasern, Partikeln oder Flächengebilden, insbesondere zur Ausrüstung oder Imprägnierung von Textilien, zur Herstellung von Papiertüchern, bei der Beschichtung von Füllstoffen; zur Herstellung von Reinigungs- und Pflegeformulierungen für den Haushalt oder für industrielle Anwendungen, insbesondere zur Herstellung von Weichspülmitteln; zur Herstellung von kosmetischen, pharmazeutischen und dermatologischen Zusammensetzungen, insbesondere kosmetischen Reinigungs- und Pflegeformulierungen, Haarbehandlungsmitteln und Haarnachbehandlungsmitteln; zur Reinigung und Pflege von harten Oberflächen; als Prozesshilfsmittel bei der Extrusion von Thermoplasten; zur Herstellung von thermoplastischen Formkörpern und/oder als Adjuvant im Pflanzenschutz; zur Herstellung von Baustoffzusammensetzungen; zur Herstellung von silikonhaltigen Beschichtungen, insbesondere Silikontrennbeschichtungen.

Desweiteren gelangt man unter Umsetzung der endäquilibrierten linearen α,ω-Acetoxygruppen-tragende Siloxane mit Polyetherolen, Polyetherdiolen und/oder Monoolen, wobei die Umsetzung in Gegenwart mindestens einer Base, insbesondere in Gegenwart von Carbonatsalzen, Ammoniak oder eines organischen Amins erfolgt, und wobei die Umsetzung vorzugsweise im Temperaturbereich von 40 bis 180°C, bevorzugt zwischen 50 und 160°C, besonders bevorzugt zwischen 80 bis 150°C durchgeführt wird, zu den angestrebten SiOC-verknüpften linearen Polyethersiloxanen.

Vorzugsweise kann der Austausch der Siloxan gebundenen Acetoxygruppen durch die Umsetzung mit Polyetherolen, Polyetherdiolen und/oder Monoolen unter Verwendung eines inerten Lösungsmittels, bevorzugt unter Verwendung eines inerten und zugleich mit entstehender und gegebenenfalls bereits vorhandener Essigsäure Azeotrop bildenden Lösungsmittels erfolgen, wobei das inerte Lösungsmittel vorteilhafterweise ein aromatisches, bevorzugt alkylaromatisches Lösungsmittel ist, und ganz besonders bevorzugt ausgewählt aus Toluol, Xylol und Ester ausgewählt aus Methoxypropylacetat, Ethyl- oder Butylacetat.

Die Umsetzung kann in einem Reaktor erfolgen, dessen Volumen mindestens 1 Liter, vorzugsweise mindestens 5 Liter, insbesondere mindestens 10 Liter beträgt, und vorzugsweise maximal 500000 Liter beträgt.

Bevorzugt kann der Austausch der Siloxan gebundenen Acetoxygruppen durch die Umsetzung mit Polyetherolen, Polyetherdiolen und /oder Monoolen lösemittelfrei erfolgen, d.h. ohne Zusatz von in der Reaktion inerten Hilfslösungsmitteln. Alle Verbindungen mit alkoholischen OH-Gruppen (Polyetherole, Polyetherdiole und /oder Monoole) sind hingegen Reaktanden.

Als Polyetherol können solche der Formel (II)

A[-O-(CH₂-CHR'-O-)ₘ-(CH₂-CH₂-O-)ₙ--(CH₂-CH(CH₃)-O-)o-Z]ₐ (II)

mit
- A: entweder Wasserstoff oder ein mindestens ein Kohlenstoffatom aufweisender gesättigter oder ungesättigter organischer Rest, bevorzugt ein mindestens ein Kohlenstoffatom aufweisender organischer Rest einer organischen Startverbindung zur Bereitung der Verbindung, besonders bevorzugt eine Methyl-, Ethyl-, Propyl-,iso-Propyl-, Butyl-, isoButyl-, Vinyl- oder Allylgruppe ist,
- R': unabhängig voneinander eine gesättigte Alkylgruppe mit 2-18 C-Atomen ist oder ein aromatischer Rest, respektive bevorzugt eine Ethylgruppe oder ein Phenylrest,
- Z: Wasserstoff, gleich 0 bis zu 50, bevorzugt 0 bis zu 30, besonders bevorzugt 0 bis zu 20 ist
- m n: gleich 0 bis zu 250, bevorzugt 3 bis zu 220, besonders bevorzugt 5 bis zu 200 ist
- o: gleich 0 bis zu 250, bevorzugt 3 bis zu 220, besonders bevorzugt 5 bis zu 200 ist
- a: gleich 1 bis zu 8, bevorzugt größer 1 bis zu 6, besonders bevorzugt 1, 2, 3 oder 4,
mit der Maßgabe, dass die Summe aus m, n und o gleich oder größer als 1 ist und mit der Maßgabe, dass mindestens A oder Z Wasserstoff repräsentieren, eingesetzt werden.

Die Monoole können aus Ethanol, Propanol, Isopropanol, Butanol, Isobutanol und Polyetherol gemäß der Formel (II) ausgewählt werden, wobei A dann nicht Wasserstoff entspricht.

Man kann mindestens 1 Mol an Polyether gebundene OH-Funktionalität pro Mol Acetoxygruppe des Siloxans, bevorzugt 1 bis 2 Mol an Polyether gebundene OH-Funktionalität, bevorzugt 1,1 bis 1,6 Mol an Polyether gebundene OH-Funktionalität, besonders bevorzugt 1,2 bis 1,4 Mol an Polyether gebundene OH-Funktionalität pro Mol Acetoxygruppe des Siloxans einsetzen.

Die Reaktion der linearen α,ω-Diacetoxypolydimethylsiloxane mit Polyetherolen, Polyetherdiolen und/oder Monoolen wird in einem unter Reaktionsbedingungen inerten Lösungsmittel durchgeführt, wobei bevorzugte Lösungsmittel Toluol und/oder die reinen oder als Isomerengemisch vorliegenden Xylole sind, und wobei diese Lösungsmittel bevorzugt in Gesamtmengen von 5 bis 35 Gew.-%, vorzugsweise 10 bis 35 Gew.-%, bezogen auf die Masse der Reaktionsmatrix eingesetzt werden, und wobei der Gesamtwassergehalt der Lösungsmittel ≤ 50 Massen-ppm, vorzugsweise ≤ 25 Massen-ppm, besonders bevorzugt ≤ 10 Massen-ppm beträgt, wobei die Bestimmung des Wassergehaltes durch Titration nach Karl Fischer erfolgt.

Die Reaktion der linearen α,ω-Diacetoxypolydimethylsiloxane mit Polyetherolen, Polyetherdiolen und/oder Monoolen wird im Temperaturbereich von 40 bis 180°C, bevorzugt zwischen 50 und 160°C, besonders bevorzugt zwischen 80 bis 150°C durchgeführt.

Die Reaktion der linearen α,ω-Diacetoxypolydimethylsiloxane wird mit Polyetherolen, Polyetherdiolen und/oder Monoolen bei vermindertem Druck und/oder unter Durchleiten eines Inertgases durchgeführt.

Die supersauren (vorzugsweise trifluormethansulfonsauren) (end)äquilibrierten linearen α,ω-Diacetoxy-polydimethylsiloxane können mit Polyetherolen, Polyetherdiolen und/oder Monoolen durch Hinzufügen einer festen, flüssigen oder gasförmigen Base zur Umsetzung gebracht werden, gegebenenfalls unter Einsatz inerter Lösungsmittel. Einzusetzende einfache Basen sind zum Beispiel Alkali- bzw. Erdalkali-Carbonate und/ oder -Hydrogencarbonate und/ oder gasförmiger Ammoniak und/ oder Amine. Der bekannten Kondensationsneigung von Acetoxysiloxanen Rechnung tragend, sind dabei ganz besonders bevorzugt solche Basen, die auf Grund ihrer chemischen Zusammensetzung kein Wasser in das Reaktionssystem eintragen. Somit haben wasserfreie Carbonate vor Hydrogencarbonaten und Hydratwasser-freie vor Hydratwasserenthaltenden Basen jeweils den Vorzug.

Die Schwerlöslichkeit der Alkali- bzw. Erdalkali-Carbonate und/ oder -Hydrogencarbonate im Reaktionssystem berücksichtigend, wählt man von diesen höhere Überschüsse, die vorzugsweise mindestens dem 2000-fachen stöchiometrischen Äquivalent der im α,ω-Diacetoxypolydimethylsiloxan enthaltenen Supersäure (vorzugsweise Trifluormethansulfonsäure) entsprechen.

Ganz besonders bevorzugt ist die Verwendung gasförmigen Ammoniaks als Base, so dass die bei der Reaktion freiwerdende Essigsäure als Ammoniumacetat gebunden wird.

Die Menge der in das Reaktionssystem eingetragenen festen, flüssigen oder gasförmigen Base wird so bemessen, dass sie sowohl für die Neutralisation der im System vorhandenen Supersäure (vorzugsweise Trifluormethansulfonsäure), als auch der Salzfällung der am Siloxan gebundenen Acetatgruppen sowie der Fällung des noch im Reaktionssystem vorhandenen Acetanhydrids sowie gegebenenfalls freier Essigsäure dient. Die Reaktion wird vorzugsweise bei Temperaturen zwischen 20 bis 120°C, vorzugsweise zwischen 20 und 70°C über die Dauer von 1 bis 10, vorzugsweise mindestens über die Dauer von 1 bis 3 Stunden durchgeführt.

Man kann das supersaure (vorzugsweise trifluormethansulfonsaure) endäquilibrierte lineare α,ω-Diacetoxy-polydimethylsiloxan mit Polyetherolen, Polyetherdiolen und/ oder Monoolen bei Temperaturen von < 25°C unter Rühren vorlegen und dann Ammoniak einleiten. Diese unter starkem Ammoniakeinsatz gefahrene Ausgestaltungsvariante bindet neben im Reaktionssystem vorhandener Supersäure (vorzugsweise Trifluormethansulfonsäure), Acetanhydrid und gegebenenfalls freier Essigsäure die bei der Reaktion freiwerdende Essigsäure als Ammoniumacetat. Die Reaktion wird vorzugsweise bei Temperaturen zwischen 20 und 70°C über die Dauer von vorzugsweise 1 bis 3 Stunden durchgeführt.

Werden die erfindungsgemäß hergestellten supersauren (vorzugsweise trifluormethansulfonsauren), (end)äquilibrierten linearen α,ω-Diacetoxypolydimethylsiloxane mit Polyetherdiolen (Formel (II) mit A = Wasserstoff) durch Hinzufügen einer festen, flüssigen oder gasförmigen Base zur Umsetzung gebracht, bevorzugt unter Verwendung eines geeigneten Lösungsmittels, so gelangt man zu linearen A(BA)n-Polyethersiloxanstrukturen, die insbesondere eine herausragende Bedeutung als Polyurethanschaumstabilisatoren für viskoelastische PU-Schäume und für den sogenannten PU-Schlagschaum (zum Beispiel für die Teppichrückseitenbeschichtung) haben.

Von entscheidender Bedeutung für das Erreichen einer hochmolekularen SiOC-verknüpften A(BA)n-Polyethersiloxanstruktur erweist sich die Qualität des eingesetzten, supersauren (vorzugsweise trifluormethansulfonsauren) linearen α,ω-Diacetoxypolydimethylsiloxans. Wie die Erfinder im Rahmen einer breit angelegten Untersuchung überraschend festgestellt haben, ist die Gewährleistung eines perfekten Äquilibrierergebnisses im α,ω-Diacetoxypolydimethylsiloxan ( = end-äquilibriert) für den Aufbau hochmolekularer SiOC-verknüpfter A(BA)n-Polyethersiloxanstruktur erforderlich.

Für den Fachmann unvorhersehbar werden auf diese Weise Strukturen erhalten, die als Stabilisatoren bei der Herstellung von Polyurethanschäumen (PU-Schäume), insbesondere PU-Weichschäumen, sprunghaft bessere Eigenschaften aufweisen.

Ebenfalls interessant ist daher ein Verfahren zur Herstellung von SiOC-verknüpften, linearen Polydimethylsiloxan-Polyoxyalkylen-Blockcopolymeren mit sich wiederholenden (AB)-Einheiten durch Umsetzung von Polyetherdiolen mit supersauren (vorzugsweise trifluormethansulfonsauren) endäquilibrierten linearen α,ω-Diacetoxypolydimethylsiloxanen, wobei man die Umsetzung durch Hinzufügen einer festen, flüssigen oder gasförmigen Base vornimmt und das gegebenenfalls unter Einsatz inerter Lösungsmittel.

Äquilibrierte lineare α,ω-Diacetoxypolydimethylsiloxane dieser Qualität, also endäquilibrierte α,ω-Diacetoxypolydimethylsiloxane, können sehr vorteilhaft, das heißt auch nach sehr kurzer Reaktionszeit, durch die Umsetzung von Hydroxygruppen tragenden Siloxanen mit Acetanhydrid in Gegenwart von Supersäure (vorzugsweise Trifluormethansulfonsäure) und Essigsäure hergestellt werden. Essigsäure wird dabei vorzugsweise in Mengen von 0,4 bis 3,5 Gewichtsprozent, bevorzugt 0,5 bis 3 Gewichtsprozent, weiter bevorzugt 0,8 bis 1,8 Gewichtsprozent, besonders bevorzugt in Mengen von 1,0 bis 1,5 Gewichtsprozent bezogen auf die Reaktionsmatrix umfassend Acetanhydrid und Hydroxygruppen tragende Siloxane, hinzugesetzt.

Die Bereitstellung von erfindungsgemäß einsetzbaren trifluormethansulfonsauren, endäquilibrierten linearen α,ω-Diacetoxypolydimethylsiloxanen wird exemplarisch in Beispiel 1 der vorliegenden Erfindung beschrieben.

Erfindungsgemäß bewährt hat sich eine Reaktionsverfolgung, bei der man über die Reaktionszeit hinweg Proben aus der Reaktionsmatrix zieht, die man dann zum Beispiel mit Hilfe der ²⁹Si-NMR und/ oder ¹³C-NMR-Spektroskopie analysiert. Die Abnahme des Integrals der für die Präsenz von Acetoxydimethylsiloxygruppen -OSi(CH₃)₂OCOCH₃ charakteristischen Signallagen geht mit der beabsichtigten SiOC-Verknüpfung zum angestrebten Polyethersiloxan-Copolymer einher und ist ein verlässlicher Indikator für den erzielten Reaktionsumsatz.

Die erfindungsgemäß hergestellten linearen Siliconpolyether-Copolymeren eignen sich allein und oder im Gemisch mit anderen Komponenten zur Herstellung von Zubereitungen für Entschäumer, Entlüfter, Schaumstabilisatoren, Netzmittel, Lack- und Verlaufsadditive oder als Dismulgatoren.

Des Weiteren eignen sich die erfindungsgemäß hergestellten linearen Siliconpolyether-Copolymeren zur Herstellung von Dieselentschäumern, von Hydrophobierungsmitteln, von Polymerdispersionen, von Klebstoffen oder Dichtstoffen, von Papiertüchern; von Reinigungs- und Pflegeformulierungen für den Haushalt oder für industrielle Anwendungen, insbesondere zur Herstellung von Weichspülmitteln, von kosmetischen, pharmazeutischen und dermatologischen Zusammensetzungen, insbesondere kosmetischen Reinigungs- und Pflegeformulierungen, Haarbehandlungsmitteln und Haarnachbehandlungsmitteln; von Baustoffzusammensetzungen, von thermoplastischen Formkörpern.

Auch die Verwendung der erfindungsgemäßen Zubereitung als Prozesshilfsmittel bei der Extrusion von Thermoplasten, als Adjuvant im Pflanzenschutz, als Additiv zur Reinigung und Pflege von harten Oberflächen, zur Oberflächenbehandlung von Fasern, Partikeln oder Flächengebilden, insbesondere zur Ausrüstung oder Imprägnierung von Textilien, oder bei der Beschichtung von Füllstoffen ist vorstellbar, ebenso zur Herstellung von silikonhaltigen Beschichtungen, insbesondere Silikontrennbeschichtungen.

### Beispiele:

Die folgenden Beispiele dienen dem Fachmann allein zur Erläuterung dieser Erfindung und stellen keinerlei Beschränkung des beanspruchten Gegenstandes dar. Die Bestimmung der Wassergehalte erfolgt grundsätzlich mit der Karl-Fischer-Methode in Anlehnung an DIN 51777, DGF E-III 10 und DGF C-III 13a. Die ²⁹Si-NMR-Spektroskopie diente in allen Beispielen der Reaktionsverfolgung.

Die ²⁹Si-NMR-Proben werden im Rahmen dieser Erfindung bei einer Messfrequenz von 79,49 MHz in einem Bruker Avance III Spektrometer, das mit einem Probenkopf 287430 mit 10 mm Spaltbreite ausgestattet ist, bei 22°C gelöst in CDCl3 und gegen Tetramethylsilan (TMS) als externem Standard [δ(²⁹Si) = 0,0 ppm] gemessen.

Die GPC's (Gel-Permeations-Chromatographie) werden unter Verwendung von THF als mobiler Phase an einer Säulenkombination SDV 1000/10000A, Länge 65 cm, ID 0,80 bei einer Temperatur von 30°C an einem SECcurity² GPC System 1260 (PSS Polymer Standards Service GmbH) aufgenommen.

Die Gaschromatogramme werden an einem GC-Gerät des Typs GC 7890B der Fa. Agilent Technologies ausgestattet mit einer Säule vom Typ HP-1; 30m x 0,32mm ID x 0,25µm dF (Agilent Technologies Nr. 19091Z-413E) und Wasserstoff als Trägergas mit folgenden Parametern aufgenommen:
Detektor: FID; 310°C
Injektor: Split; 290°C
Mode: constant flow 2 mL/min
Temperaturprogramm: 60°C mit 8°C/min -150°C mit 40°C/min - 300°C 10 min.

Als Indikator für das Erreichen des Äquilibriergleichgewichtes wird der gaschromatographisch bestimmte Gesamtcyclengehalt definiert als die Summe der D4-, D5-, D6-Gehalte bezogen auf die Siloxanmatrix und festgestellt nach der Derivatisierung der α,ω-Diacetoxypolydimethylsiloxane zu den entsprechenden α,ω-Diisopropoxypolydimethylsiloxanen herangezogen. Die Derivatisierung zu den α,ω-Diisopropoxypolydimethylsiloxanen wird hierbei bewusst gewählt, um eine unter den Bedingungen der gaschromatographischen Analyse gegebenenfalls erfolgende, thermisch induzierte Rückspaltungsreaktion der α,ω-Diacetoxy-polydimethylsiloxane zu verhindern (zur Rückspaltungsreaktion siehe u.a. J. Pola et al., Collect. Czech. Chem. Commun. 1974, 39(5), 1169-1176 und auch W. Simmler, Houben-Weyl, Methods of Organic Chemistry, Vol. VI/2, 4th Edition, O-Metal Derivates of Organic Hydroxy Compounds S. 162 ff)).

### Beispiel 1 (erfindungsgemäß)

Herstellung eines Acetoxy-terminierten, linearen Polydimethylsiloxans mit 3,0% Essigsäure-Zusatz

In einem 1000-ml-Vierhalskolben mit KPG-Rührer, Innenthermometer und aufgesetztem Rückflusskühler werden 77,3 g (0,757 mol) Essigsäureanhydrid zusammen mit 732,8 g (0,267 mol) eines α,ω-Dihydroxypolydimethylsiloxans (M: 2742 g/ mol und 24,3 g Essigsäure (3,0 Gew.-% bezogen auf die Gesamtmasse der Reaktanden) unter Rühren vorgelegt und mit 1,62 g (0,88 ml) Trifluormethansulfonsäure (0,2 Gewichtsprozent bezogen auf den Gesamtansatz) versetzt und zügig auf 150°C erhitzt. Die zu Beginn leicht trübe Reaktionsmischung wird unter weiterem Rühren für 4 Stunden bei dieser Temperatur belassen.

Nach Erkalten des Ansatzes wird eine farblos-klare, leichtbewegliche Flüssigkeit isoliert, deren ²⁹Si-NMR-Spektrum die Präsenz von Si-Acetoxygruppen in einer Ausbeute von ca. 93% bezogen auf eingesetztes Essigsäureanhydrid belegt entsprechend einem α,ω-Diacetoxypolydimethylsiloxan mit einer mittleren Gesamtkettenlänge von ca. 14.

Überführung des linearen α,ω-Diacetoxypolydimethylsiloxans in das entsprechende α,ω-Diisopropoxy-polydimethylsiloxan zur analytischen Charakterisierung.

Unmittelbar nach der Synthese werden 50,0 g dieses trifluormethansulfonsauren, äquilibrierten α,ω-Diacetoxypolydimethylsiloxans in einem 250-ml-Vierhalsrundkolben ausgerüstet mit mit KPG-Rührer, Innenthermometer und aufgesetztem Rückflusskühler zusammen mit 11,3 g eines über Molekularsieb getrockneten Isopropanols unter Rühren bei 22°C vermischt. Die Reaktionsmischung wird dann durch Einleiten gasförmigen Ammoniaks (NH₃) bis zur alkalischen Reaktion (feuchtes Universalindikatorpapier) beaufschlagt und dann noch 45 Minuten bei dieser Temperatur nachgerührt. Die ausgefallenen Salze werden mit Hilfe eines Faltenfilters abgetrennt.

Isoliert wird eine farblos, klare Flüssigkeit deren begleitendes ²⁹Si-NMR-Spektrum die quantitative Umwandlung des α,ω-Diacetoxypolydimethylsiloxans in ein α,ω-Diisopropoxypolydimethylsiloxan belegt.

Ein Aliquot dieses α,ω-Diisopropoxypolydimethylsiloxans wird entnommen und gaschromatographisch analysiert. Das Gaschromatogramm weist folgende Gehalte (Angaben in Gewichtsprozent) auf:

| D₄ | D₅ | D₆ | Summe (D₄ - D₆) | Isopropanolgehalt |
|---|---|---|---|---|
| 4,12 % | 2,61 % | 0,84 % | 7,57 % | 4,58 % |

Unter Berücksichtigung des Isopropanolüberschusses sind hierbei die Gehalte an Siloxancyclen (D₄, D₅ und D₆) allein bezogen auf den Siloxananteil berechnet.

## Patentansprüche

1. Verfahren zur Herstellung supersaurer, insbesondere trifluormethansulfonsaurer, (end)äquilibrierter linearer α,ω-Acetoxygruppen-tragender Siloxane, **dadurch gekennzeichnet, dass** man
(i) lineare α,ω-Hydroxygruppen tragende Siloxane,
(ii) unter Einsatz von Supersäure, besonders bevorzugt Perfluoralkansulfonsäure, insbesondere bevorzugt Trifluormethansulfonsäure als Katalysator,
(iii) mit Acetanhydrid und unter Zusatz von Essigsäure
umsetzt.

2. Verfahren nach Anspruch 1, **dadurch gekennzeichnet, dass** die neben der Essigsäure eingesetzte Säure Supersäure ist, die einen pKa-Wert kleiner -3,0 aufweist, vorzugsweise fluorierte und/oder perfluorierte Sulfonsäure, Fluorsulfonsäure HSO₃F, Fluor-Antimonsäure HSbF₆ und/oder Perfluorbutansulfonsäure C₄F₉SO₃H und ganz besonders bevorzugt Trifluormethansulfonsäure CF₃SO₃H.

3. Verfahren nach Anspruch 1 oder 2, **dadurch gekennzeichnet, dass** die linearen α,ω-Hydroxygruppen tragenden Siloxane der Formel (I) genügen: 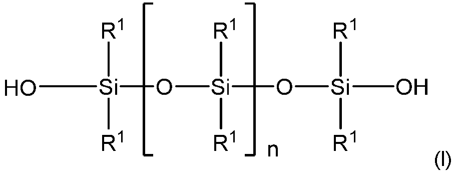 mit R¹, unabhängig voneinander, gleich Alkylrest und/oder aromatischer Rest, umfassend 1 bis 10 C-Atome, bevorzugt Methylrest, und mit 1 ≤ n ≤ 19.000, bevorzugt n zwischen 3 und 200, besonders bevorzugt n zwischen 20 und 100.

4. Verfahren nach zumindest einem der Ansprüche 1 bis 3, **dadurch gekennzeichnet, dass** die Essigsäure in Mengen von 0,4 bis 3,5 Gewichtsprozent, vorzugsweise 0,5 bis 3 Gewichtsprozent, bevorzugt 0,8 bis 1,8 Gewichtsprozent, besonders bevorzugt in Mengen von 1,0 bis 1,5 Gewichtsprozent bezogen auf die Reaktionsmatrix, umfassend Acetanhydrid und Hydroxygruppen tragende Siloxane, hinzugefügt wird, sowie, dass neben der Essigsäure noch Supersäure, besonders bevorzugt Perfluoralkansulfonsäure und insbesondere Trifluormethansulfonsäure in Mengen von 0,1 bis 1,0 Gewichtsprozent, bevorzugt 0,1 bis 0,3 Gewichtsprozent, bezogen auf die Reaktionsmatrix, umfassend Acetanhydrid und Hydroxygruppen tragenden Siloxanen, eingesetzt wird.

5. Verfahren nach zumindest einem der Ansprüche 1 bis 4, **dadurch gekennzeichnet, dass** die einzusetzende Acetanhydridmenge mindestens so zu bemessen ist, das alle Si-gebundenen Hydroxygruppen des eingesetzten α,ω-Hydroxygruppen tragenden Siloxans durch Acetoxygruppen ersetzt werden und gleichzeitig das freigesetzte Wasseräquivalent durch Reaktion mit weiterem Acetanhydrid in Form von zwei Äquivalenten Essigsäure gebunden wird.

6. Verfahren nach zumindest einem der Ansprüche 1 bis 5, **dadurch gekennzeichnet, dass** die Umsetzung in einem Reaktor erfolgt, dessen Volumen mindestens 1 Liter, vorzugsweise mindestens 5 Liter, insbesondere mindestens 10 Liter beträgt, und vorzugsweise maximal 500000 Liter beträgt.

7. Verfahren nach zumindest einem der Ansprüche 1 bis 6, **dadurch gekennzeichnet, dass** die supersauren, insbesondere trifluormethansulfonsauren, (end)äquilibrierten linearen α,ω-Acetoxygruppen-tragenden Siloxane zumindest 3, vorzugsweise 5 bis 50, bevorzugt 7 bis 25, besonders bevorzugt 10 bis 20 Organosiloxaneinheiten aufweisen.

8. Supersaure, insbesondere trifluormethansulfonsaure, (end)äquilibrierte lineare α,ω-Acetoxygruppen-tragende Siloxane, hergestellt nach einem Verfahren nach einem der Ansprüche 1 bis 7, **dadurch gekennzeichnet, dass** sie Gesamtcyclengehalte, definiert als Summe der Gehaltsanteile der zyklischen Siloxane umfassend D₄, D₅ und D₆ bezogen auf die Siloxanmatrix und gaschromatographisch ermittelt nach deren Derivatisierung zu den entsprechenden linearen α,ω-Isopropoxysiloxanen von kleiner 13, vorzugsweise kleiner 12 Gewichtsprozent aufweisen, und wobei vorzugsweise gilt, dass die supersauren, insbesondere trifluormethansulfonsauren, (end)äquilibrierten linearen α,ω-Acetoxygruppen-tragenden Siloxane zumindest 3, vorzugsweise 5 bis 50, bevorzugt 7 bis 25, besonders bevorzugt 10 bis 20 Organosiloxaneinheiten aufweisen.

## Claims

1. Process for producing superacid-acidified, in particular trifluoromethanesulfonic acid-acidified, (end-)equilibrated linear α,ω-acetoxy-bearing siloxanes, **characterized in that**
(i) linear α,ω-hydroxy-bearing siloxanes,
(ii) using superacid, particularly preferably perfluoroalkanesulfonic acid, especially preferably trifluoromethanesulfonic acid, as catalyst,
(iii) are reacted with acetic anhydride and with addition of acetic acid.

2. Process according to Claim 1, **characterized in that** the acid employed in addition to the acetic acid is a superacid having a pKa of less than -3.0, preferably fluorinated and/or perfluorinated sulfonic acid, fluorosulfonic acid HSO₃F, fluoroantimonic acid HSbF₆ and/or perfluorobutanesulfonic acid C₄F₉SO₃H and very particularly preferably trifluoromethanesulfonic acid CF₃SO₃H.

3. Process according to Claim 1 or 2, **characterized in that** the linear α,ω-hydroxy-bearing siloxanes satisfy formula (I): 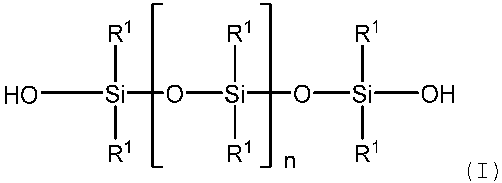 where R¹ is independently at each occurrence an alkyl radical and/or aromatic radical comprising 1 to 10 carbon atoms, preferably a methyl radical, and where 1 ≤ n ≤ 19 000, preferably n is between 3 and 200, particularly preferably n is between 20 and 100.

4. Process according to at least one of Claims 1 to 3, **characterized in that** the acetic acid is added in amounts of 0.4 to 3.5 per cent by weight, by preference 0.5 to 3 per cent by weight, preferably 0.8 to 1.8 per cent by weight, particularly preferably in amounts of 1.0 to 1.5 per cent by weight, based on the reaction matrix comprising acetic anhydride and hydroxy-bearing siloxanes, and **in that** in addition to the acetic acid further superacid, particularly preferably perfluoroalkanesulfonic acid and in particular trifluoromethanesulfonic acid, is employed in amounts of 0.1 to 1.0 per cent by weight, preferably 0.1 to 0.3 per cent by weight, based on the reaction matrix comprising acetic anhydride and hydroxy-bearing siloxanes.

5. Process according to at least one of Claims 1 to 4, **characterized in that** the acetic anhydride amount to be employed has to be at least sufficient to ensure that all Si-bonded hydroxy groups of the employed α,ω-hydroxy-bearing siloxane are replaced by acetoxy groups while at the same time the liberated water equivalent is bound in the form of two equivalents of acetic acid by reaction with further acetic anhydride.

6. Process according to at least one of Claims 1 to 5, **characterized in that** the reaction is carried out in a reactor having a volume of at least 1 litre, preferably at least 5 litres, in particular at least 10 litres and preferably not more than 500 000 litres.

7. Process according to at least one of Claims 1 to 6, **characterized in that** the superacid-acidified, in particular trifluoromethanesulfonic acid-acidified, (end-)equilibrated linear α,ω-acetoxy-bearing siloxanes have at least 3, preferably 5 to 50, preferably 7 to 25, particularly preferably 10 to 20, organosiloxane units.

8. Superacid-acidified, in particular trifluoromethanesulfonic acid-acidified, (end-)equilibrated linear α,ω-acetoxy-bearing siloxanes produced by a process according to any of Claims 1 to 7, **characterized in that** they have total cycles contents defined as the sum of the content fractions of the cyclic siloxanes comprising D₄, D₅ and D₆ based on the siloxane matrix and determined by gas chromatography after their derivatization to afford the corresponding linear α,ω-isopropoxysiloxanes of less than 13, preferably less than 12, per cent by weight, and wherein it is preferably the case that the superacid-acidified, in particular trifluoromethanesulfonic acid-acidified, (end-)equilibrated linear α,ω-acetoxy-bearing siloxanes have at least 3, preferably 5 to 50, preferably 7 to 25, particularly preferably 10 to 20, organosiloxane units.

## Revendications

1. Procédé de préparation de siloxanes superacides, en particulier acide trifluorométhanesulfonique, (complètement) équilibrés, linéaires, portant des groupes α,ω-acétoxy, **caractérisé en ce qu'**on transforme
(i) des siloxanes linéaires portant des groupes α,ω-hydroxy,
(ii) avec utilisation de superacide, de manière particulièrement préférée d'acide perfluoroalcanesulfonique, en particulier de préférence d'acide trifluorométhanesulfonique en tant que catalyseur,
(iii) avec de l'anhydride acétique et avec utilisation d'acide acétique.

2. Procédé selon la revendication 1, **caractérisé en ce que** l'acide utilisé en plus de l'acide acétique est un superacide qui présente une valeur pKa inférieure à -3,0, de préférence un acide sulfonique fluoré et/ou perfluoré, l'acide fluorosulfonique HSO₃F, l'acide fluoroantimonique HSbF₆ et/ou l'acide perfluorobutanesulfonique C₄F₉SO₃H et de manière tout particulièrement préférée l'acide trifluorométhanesulfonique CF₃SO₃H.

3. Procédé selon la revendication 1 ou 2, **caractérisé en ce que** les siloxanes linéaires, portant des groupes α,ω-hydroxy satisfont à la formule (I) : 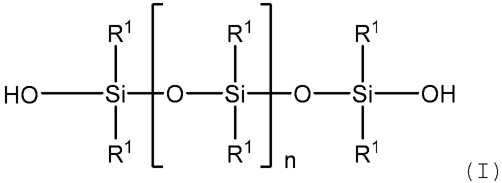 dans laquelle R¹ représente, indépendamment, un radical alkyle et/ou un radical aromatique, comprenant 1 à 10 atomes de carbone, de préférence un radical méthyle, et 1 ≤ n ≤ 19.000, de préférence n est situé entre 3 et 200, de manière particulièrement préférée n est situé entre 20 et 100.

4. Procédé selon au moins l'une des revendications 1 à 3, **caractérisé en ce que** l'acide acétique est ajouté en des quantités de 0,4 à 3,5% en poids, de préférence de 0,5 à 3% en poids, de préférence de 0,8 à 1,8% en poids, de manière particulièrement préférée de 1,0 à 1,5% en poids, par rapport à la matrice de réaction comprenant l'anhydride acétique et les siloxanes portant des groupes hydroxy, et **en ce que**, en plus de l'acide acétique, on utilise encore un superacide, de manière particulièrement préférée un acide perfluoroalcanesulfonique et en particulier l'acide trifluorométhanesulfonique en des quantités de 0,1 à 1,0% en poids, de préférence de 0,1 à 0,3% en poids, par rapport à la matrice de réaction comprenant l'anhydride acétique et les siloxanes portant des groupes hydroxy.

5. Procédé selon au moins l'une des revendications 1 à 4, **caractérisé en ce que** la quantité d'anhydride acétique à utiliser est au moins dimensionnée de manière telle que tous les groupes hydroxy liés à Si du siloxane portant des groupes α,ω-hydroxy utilisé sont remplacés par des groupes acétoxy et simultanément l'équivalent d'eau libéré est lié par réaction avec de l'anhydride acétique supplémentaire sous forme de deux équivalents d'acide acétique.

6. Procédé selon au moins l'une des revendications 1 à 5, **caractérisé en ce que** la transformation a lieu dans un réacteur dont le volume représente au moins 1 litre, de préférence au moins 5 litres, en particulier au moins 10 litres et de préférence au maximum 500.000 litres.

7. Procédé selon au moins l'une des revendications 1 à 6, **caractérisé en ce que** les siloxanes superacides, en particulier acide trifluorométhanesulfonique, (complètement) équilibrés, linéaires, portant des groupes α,ω-acétoxy présentent au moins 3, de préférence 5 à 50, de préférence 7 à 25, de manière particulièrement préférée 10 à 20 motifs organosiloxane.

8. Siloxanes superacides, en particulier acide trifluorométhanesulfonique, (complètement) équilibrés, linéaires, portant des groupes α,ω-acétoxy, préparés selon un procédé selon l'une des revendications 1 à 7, **caractérisés en ce qu'**ils présentent des teneurs totales en cycles, définies comme la somme des proportions des siloxanes cycliques comprenant D₄, D₅ et D₆, par rapport à la matrice de siloxane et déterminées par chromatographie en phase gazeuse après leur dérivation en α,ω-isopropoxysiloxanes linéaires correspondants, inférieures à 13, de préférence inférieures à 12% en poids et, de préférence, **en ce que** les siloxanes superacides, en particulier acide trifluorométhanesulfonique, (complètement) équilibrés, linéaires, portant des groupes α,ω-acétoxy présentent au moins 3, de préférence 5 à 50, de préférence 7 à 25, de manière particulièrement préférée 10 à 20 motifs organosiloxane.
